# EUROPEAN PATENT APPLICATION

(11) **EP 4 032 516 A1**
(43) Date of publication of application: **27.07.2022**
(21) Application number: 20865676.9
(22) Date of filing: 17.09.2020
(51) Int. Cl.: A61F 13/47, A61F 13/475, A61F 13/476, A61F 13/53, A61F 13/533, A61F 13/536

(54) **ABSORBENT ARTICLE**

(30) Priority: 19.09.2019 JP 2019170372
(71) Applicant: DAIO PAPER CORPORATION, Shikokuchuo-shi Ehime 799-0492 (JP)
(72) Inventor: SUYAMA, Junnosuke, Sakura-shi, Tochigi 329-1411 (JP)
(74) Representative: Schön, Christoph
(86) International application number: PCT/JP2020/035262
(87) International publication number: WO 2021/054392

(57) **Abstract**

[Problem to be solved] To reduce uncomfortable feeling during wearing, and to prevent leakage from a side portion.

[Solution] An absorber 4, and side sheets 7 disposed on both side portions in a longitudinal direction of a skin-contact surface side over an entire length in the longitudinal direction are provided. The side sheets 7 do not include an elastically stretchable member. There are provided a non-adhesive portion 10, in which a center portion in the width direction of the side sheets 7 is not bonded to the absorber 4 side, in a longitudinal section including a region corresponding to a body fluid discharge portion H of a wearer, and adhesive portions 11 and 12 in which side sheets 7 are bonded to the absorber 4 at an outer side in the width direction and at both end sides in the longitudinal direction of the non-adhesive portion 10. An edge of side sheet 7 in the center side in the width direction is located to the center side in the width direction from a side edge of a narrowest portion 4a of the absorber 4 in the longitudinal section including a region corresponding to a body fluid discharge portion H of a wearer. An edge in the center side in the width direction of the adhesive portion 11 adjacent to the outer side of the non-adhesive portion 10 in the width direction is provided in a position overlapped to or in the vicinity of the side edge of a narrowest portion 4a of the absorber 4.

## Description

### TECHNICAL FIELD

The present invention relates to an absorbent article mainly such as sanitary napkins, vaginal sheets, incontinence pads, and toiletries, and more particularly to an absorbent article configured not to include an elastically stretchable member in side sheets in order to reduce uncomfortable feeling during wearing.

### BACKGROUND ART

Conventionally, as the absorbent article, there is known an absorbent article including a liquid-impermeable back-surface sheet of a polyethylene sheet, a polyethylene sheet-laminated nonwoven fabric, or the like, a liquid-permeable top-surface sheet of a non-woven fabric or permeable plastic sheet, and an absorber made of cotton pulp or the like, interposed between the back-surface sheet and the top-surface sheet.

In particular, in the sanitary napkin for night use, in order to prevent lateral leakage of a body fluid such as menstrual blood flowing in the width direction and leaking from the side edge portion during wearing, there have been proposed a large number of sanitary napkins having three-dimensional gathers standing up toward the skin side by contraction force of an elastically stretchable member formed at the skin contact surface side on both sides in the longitudinal direction, respectively.

For example, the following Patent Literature 1 discloses an absorbent article including a main body having an absorbent core including a narrow intermediate core portion by a pair of constricted regions, a pair of wing portions disposed at the outer side in a width direction of the constricted regions and facing each other in the width direction with the main body sandwiched therebetween, and a pair of three-dimensional guards provided on each of both sides in the width direction of a skin-contact surface, stepping over the constricted regions and extending in the longitudinal direction.

Furthermore, the following Patent Literature 2 discloses an absorbent article including a first pressing portion pressing at least a top-surface sheet and an absorbent layer in the position on both left and right sides from the center line extending in the vertical direction, and a second pressing portion provided at the outer side of the first pressing portion with a space provided between the first and second pressing portions, respectively expanding in the longitudinal direction, and also including a sheet extending in the vertical direction with a space provided in the lateral direction, on the skin-side surface, wherein the sheet is fixed to the skin side surface at a front-side portion and a back-side portion, and stands up from the skin-side surface in the intermediate portion to form a leak-proof wall, and at least one of a rising start point at the front-surface side and a rising start point at the back-surface side of the leak-proof wall is located in the vicinity of the second pressing portion.

### Citation List

### Patent Literature

Patent Literature 1: Japanese Unexamined Patent Application, Publication No. 2019-103711
Patent Literature 2: Japanese Unexamined Patent Application, Publication No. 2004-154154

### SUMMARY OF THE INVENTION

### Technical Problems

However, in the three-dimensional guard and the leak-proof wall described in the Patent Literatures 1 and 2, since the side sheet stands up toward the skin side by the contraction force of the elastically stretchable member, there may be a risk that the contraction force of the elastically stretchable member acts on the skin surface of a wearer and gives uncomfortable feeling to the wearer.

On the other hand, some absorbent articles do not include an elastically stretchable member on the side sheet. However, since the side sheet does not stand up toward the skin side during wearing, leakage from the side portion easily occurs.

Then, a main problem of the present invention is to provide an absorbent article in which an elastically stretchable member is not provided in the side sheet to reduce uncomfortable feeling during wearing and to prevent leakage from the side portion.

### Solution to Problem

In order to solve the above-mentioned problem, a first embodiment provides an absorbent article including an absorber, and side sheets disposed on both side portions in a longitudinal direction on a skin-contact surface side over an entire length in the longitudinal direction, the side sheets not including an elastically stretchable member, including a non-adhesive portion, in which a center portion in the width direction of each of the side sheets is not bonded to the absorber side, in a longitudinal section including a region corresponding to a body fluid discharge portion of a wearer, and an adhesive portion in which the side sheets are bonded to the absorber side, respectively, at an outer side in the width direction and at both end sides in the longitudinal direction of the non-adhesive portion, wherein an edge of the side sheets in the center side in the width direction is located to the center side in the width direction from the side edge of the absorber, and the adhesive portion adjacent to the outer side in the width direction of the non-adhesive portion has an edge in the center side in the width direction provided in a position overlapped to or in the vicinity of a side edge of the absorber.

In the above first embodiment, since the side sheets do not include an elastically stretchable member, contraction force of the elastically stretchable member does not act on the skin surface, and as a result, uncomfortable feeling of a wearer during wearing does not occur.

Even if contraction force of the elastically stretchable member does not act on the side sheets, since the side sheets are allowed to stand up toward the skin side and prevent lateral leakage, this absorbent article includes a non-adhesive portion, in which a center portion in the width direction of the side sheets is not bonded to the absorber side, in a longitudinal section including a region corresponding to a body fluid discharge portion of a wearer, and an adhesive portion in which the side sheets are bonded to the absorber side, respectively, at an outer side in the width direction and at both end sides in the longitudinal direction of the non-adhesive portion, wherein an edge of the side sheet in the center side in the width direction is located to the center side in the width direction from the side edge of the absorber, and the adhesive portion adjacent to the outer side in the width direction of the non-adhesive portion includes an edge in the center side in the width direction provided in a position overlapped to or in the vicinity of the side edge of the absorber. Therefore, when a pressure is applied from both sides by leg pressure during wearing of the absorbent article, the both side portions of the absorber are compressed in the width direction, and thereby the both sides swell to the skin side, and the non-adhesive portion of the side sheets stands up to the skin side from the boundary portion with respect to the adjacent portion as a starting point in the outer side in the width direction. Thus, body fluids flowing toward the sides are blocked and leakage from the side portion is prevented.

A second embodiment provides an absorbent article, wherein a constricted portion obtained by constricting both side edges of the absorber inward in the width direction is formed in the longitudinal section including a region corresponding to a body fluid discharge portion of a wearer.

In the second embodiment, since the constricted portion obtained by constricting both side edges of the absorber inward in the width direction is formed in the longitudinal section including a region corresponding to a body fluid discharge portion of a wearer, leg pressure intensively acts on the narrowest portion of the constricted portion, so that the side sheet easily stands up to the skin side.

A third embodiment provides an absorbent article, wherein compressed grooves dented toward a non-skin side are formed on a skin-contact surface of a main body part in which the absorber is interposed, and wherein the compressed grooves are respectively formed on both side portions of a longitudinal section including a region corresponding to a body fluid discharge portion of a wearer respectively along the longitudinal direction and in a planar shape bulging to an outer side in the width direction.

In the above third embodiment, a planar shape of the compressed grooves is a shape along the longitudinal direction of both side portions of the longitudinal section including a region corresponding to a body fluid discharge portion of a wearer respectively and bulging to an outside in the width direction. Therefore, a leg pressure from the outer side in the width direction easily acts on the absorber of an outer side portion of the compressed groove during wearing and easily swells to the skin side of the absorber.

A fourth embodiment provides the absorbent article, wherein, in the longitudinal section including a region corresponding to a body fluid discharge portion of a wearer, a maximum bulging portion of each of the compressed grooves and a narrowest portion of the absorber are provided in a position substantially coinciding to a longitudinal direction of the absorbent article.

In the above fourth embodiment, in the absorbent article including the compressed groove, since the maximum bulging portion of each of the compressed grooves and the narrowest portion of the absorber are provided in a position substantially coinciding to the longitudinal direction of the absorbent article in the longitudinal section including a region corresponding to a body fluid discharge portion of a wearer, the leg pressure during wearing intensively acts on the absorber part between the narrowest part of the absorber and the maximum bulging portion of the compressed groove, and the absorber in this part is easily deformed, and the non-adhesive portion of the side sheets easily stands up toward the skin side.

A fifth embodiment provides the absorbent article, wherein rigidity of the absorber at the outer side in the width direction from the compressed groove is set to be lower than rigidity of the absorber between left and right compressed grooves, in the longitudinal section including a region corresponding to a body fluid discharge portion of a wearer.

In the above fifth embodiment, in the absorbent article having the compressed grooves, since rigidity of the absorber at the outer side in the width direction from the compressed groove is set to be lower than rigidity of the absorber between left and right compressed grooves in the longitudinal section including a region corresponding to a body fluid discharge portion of a wearer, an absorber in an outer side portion in the width direction from the compressed groove easily swells toward the skin side, and accordingly, the non-adhesive portion of the side sheets easily stands up toward the skin side.

A sixth embodiment provides the absorbent article, wherein a pair of wing-shaped flaps protruding to the side portions to be fixed to an undergarment are formed on both side portions in a region corresponding to a body fluid discharge portion of a wearer, a center portion in the longitudinal direction of the wing-shaped flap and a center portion in the longitudinal direction of the non-adhesive portion are provided in the position substantially coinciding with the longitudinal direction of the absorbent article.

In the above sixth embodiment, in the absorbent article including wing-shaped flaps formed on both side portions of a region corresponding to a body fluid discharge portion of a wearer, since the center portion in the longitudinal direction of the wing-shaped flap and the center portion in the longitudinal direction of the non-adhesive portion are provided in the position substantially coinciding with the longitudinal direction of the absorbent article, when the wing-shaped flap is folded such that the side edge of an undergarment is wrapped together, and fixed, the side sheets are pulled toward the outer side in the width direction, so that the non-adhesive portion in the center side in the width direction easily stands up toward the skin side.

A seventh embodiment provides absorbent article, wherein a pair of wing-shaped flaps protruding to the side portions to be fixed to an undergarment are formed on both side portions in a region corresponding to a body fluid discharge portion of a wearer, and a wing displacement prevention adhesive layer is provided on an undergarment-contact surface of the wing-shaped flaps, and the wing displacement prevention adhesive layer is disposed along a virtual line linking the side edges of the absorber in a front part and a back part of a region corresponding to a body fluid discharge portion of a wearer in the longitudinal direction.

In the above seventh embodiment, in the absorbent article provided wing-shaped flaps protruding to the side portions are formed on both side portions in a region corresponding to a body fluid discharge portion of a wearer, and the wing displacement prevention adhesive layer is provided on the undergarment-contact surface of the wing-shaped flaps, and the wing displacement prevention adhesive layer is disposed along a virtual line linking the side edges of the absorber in the front and back portions of a region corresponding to a body fluid discharge portion of a wearer in the longitudinal direction, when the wing-shaped flap is folded such that the side edge of an undergarment is wrapped together, and fixed, the vicinity of the side edge of the absorber is fixed to the undergarment by the wing displacement prevention adhesive layer, the side sheet does not easily bent, and the non-adhesive portion easily stands up.

### [Advantageous Effect of Invention]

As described above in detail, the present invention can reduce uncomfortable feeling during wearing and prevent leakage from the side part.

### BRIEF DESCRIPTION OF THE DRAWINGS

FIG. 1 is a plan view of a sanitary napkin 1 in accordance with the present invention;
FIG. 2 is an arrow view taken along the line II-II of FIG. 1;
FIG. 3 is an arrow view taken along the line III-III of FIG. 1;
FIG. 4 is an enlarged plan view of a region corresponding to a body fluid discharge portion H of a wearer;
FIG. 5(A) is a sectional view showing a state when being placed on an undergarment, and FIG. 5(B) is a sectional view showing a state when a leg pressure is applied from both sides;
FIG. 6 is an enlarged plan view showing a region corresponding to a body fluid discharge portion H of a wearer; and
FIG. 7 is an enlarged plan view showing a region corresponding to a body fluid discharge portion H of a wearer. Description of Embodiments

Hereinafter, embodiments of the present invention will be described in detail with reference to the drawings.

### [Basic Structure of Sanitary Napkin 1]

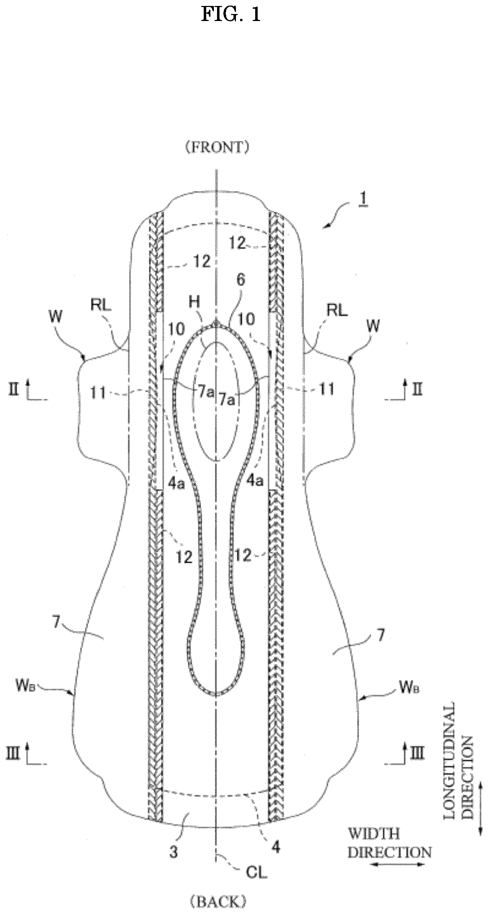

A sanitary napkin 1 according to the present invention includes, as shown in FIGS. 1 to 3, a liquid-impermeable back-surface sheet 2 composed of a polyethylene sheet and the like, a liquid-permeable top-surface sheet 3 that allows quick permeation of menstrual blood, vaginal discharge, and the like (hereinafter, which are also collectively referred to as body fluid), an absorber 4 interposed between the sheets 2 and 3 and made of a cotton-state pulp or a synthetic pulp, and side sheets 7, 7 provided on both side portions of a skin-contact surface side over substantially an entire length in the longitudinal direction. In the periphery of the absorber 4, on front and back edges, outer edges of the back-surface sheet 2 and the top-surface sheet 3 of the absorber 4 are joined with adhesives such as hot-melt or by bonding means such as heat sealing and ultrasonic sealing; on both side edge portions, the back-surface sheet 2 and the side sheets 7, which laterally protrude out of the absorber 4 are joined with adhesives such as hot-melt or by bonding means such as heat sealing and ultrasonic sealing. A pair of left and right wing flaps W and W extending outward are formed in positions on the side of the absorber substantially corresponding to a body fluid discharge portion H, and a pair of left and right back flaps W_{B} and W_{B} extending outward are formed on both sides of the back portion on the back side (buttock side) . Note here that in the example shown in the drawing, in order to keep the shape of the absorber 4 and improve dispersion, the absorber 4 is wrapped with an encapsulating sheet 5 made of crepe paper, non-woven fabric, or the like, but the encapsulating sheet 5 may not be provided. Furthermore, although not shown, a second sheet made of hydrophilic non-woven fabric having the same shape as that of the top-surface sheet 3 may be disposed adjacent to the non-skin-side of the top-surface sheet 3.

Hereinafter, the structure of the sanitary napkin 1 will be described in more detail. For the back-surface sheet 2, a sheet material having at least water-blocking property, for example, polyethylene, is used. However, from the viewpoint of preventing a moist feeling, a sheet material having moisture permeability is desirably used. As the water-blocking and moisture-permeable sheet materials, microporous sheets are suitably used. The microporous sheet is obtained by molding a sheet by melting and kneading an inorganic filler in an olefin-based resin such as polyethylene or polypropylene, followed by stretching in uniaxial or biaxial direction. On an undergarment-contact surface (outside surface) of the back-surface sheet 2, one or a plurality of stripes of sticking layers (not shown in the drawings) is formed along the longitudinal direction of the napkin, so that the sanitary napkin 1 is fixed to an undergarment when wearing on the body. As the back-surface sheet 2, a poly-laminate nonwoven fabric in which a plastic film and a nonwoven fabric are laminated may be used.

Next, for the top-surface sheet 3, porous or non-porous non-woven fabric, a porous plastic sheet, and the like, are suitably used. As the material fibers constituting the nonwoven fabric, synthetic fibers such as olefin-based fibers such as polyethylene or polypropylene, polyester-based fibers, and polyamide-based fibers, regenerated fibers such as rayon and cupra, and natural fibers such as cotton can be used, and nonwoven fabrics obtained by appropriate processing methods such as a spunlace method, a spunbond method, a thermal bond method, a meltblown method, and a needle punch method can be used. Among these processing methods, the spun lace method is excellent in being rich in flexibility, and a drape property, and the thermal bond method is excellent in bulkiness and high compression restorability. When a large number of through holes are formed in the top-surface sheet 3, the body fluid is rapidly absorbed, so that excellent dry touch property is achieved. The non-woven fabric fibers may be long fibers or short fibers, but short fibers are preferably used in order to bring out the texture of towel fabric. Furthermore, in order to fabricate embossing treatment, olefin-based fibers such as polyethylene or polypropylene having a relatively low melting point is preferably used. Furthermore, a core-sheath type fiber including a core having a high melting point and a sheath having a low melting point, a side-by-side fiber, or composite fiber of dividing fibers can be suitably used.

The absorber 4 interposed between the back-surface sheet 2 and the top-surface sheet 3 is formed of, for example, cotton pulp and water absorbing polymer. The water absorbing polymer is mixed into a pulp constituting the absorber as, for example, granular powder. Examples of the pulp include a pulp made of cellulose fibers such as chemical pulp obtained from timber or molten pulp, and artificial cellulose fibers such as rayon and acetate. From the viewpoint of function and price, softwood pulp having a fiber length longer than that of hardwood pulp is suitably used. The basis weight of the absorber 4 is set at 300 to 750 g/m², and preferably 300 to 400 g/m².

Furthermore, the absorber 4 may include synthetic fiber. For the synthetic fibers, for example, polyolefin-based fibers such as polyethylene and polypropylene, polyester-based fibers such as polyethylene terephthalate and polybutylene terephthalate, polyamide-based fibers such as nylon, and copolymers thereof may be used, and mixture of two types thereof may be used. Furthermore, a core-sheath type fiber including a core having a high melting point and a sheath having a low melting point, a side-by-side fiber, or composite fiber of dividing fibers can be suitably used. In the synthetic fiber, in order to have affinity to a body fluid, when the synthetic fiber is a hydrophobic fiber, it is desirable to use the surface that has been treated with a hydrophilizing agent.

It is preferable that a compressed groove 6 dented toward the non-skin-side is formed on the skin-contact surface of the main body part of the absorber 4. Preferably, the compressed groove is formed in both side portions of the longitudinal section including a region corresponding to a body fluid discharge portion H of a wearer along the longitudinal direction and in a planar shape bulging outward in the width direction. In an example shown in FIG. 1, the compressed groove 6 is formed in a closing curve having substantially a spoon-like shape consisting of a vertically elongated elliptical portion surrounding the region corresponding to a body fluid discharge portion H of a wearer and an elongated portion extending to a region corresponding to a buttock groove of the wearer behind the elliptical portion. However, the compressed groove may be formed by a pair of curves spaced apart in the width direction as long as the compressed groove is provided at least on both sides of the region corresponding to a body fluid discharge portion H of a wearer in the longitudinal direction.

### [Side sheet]

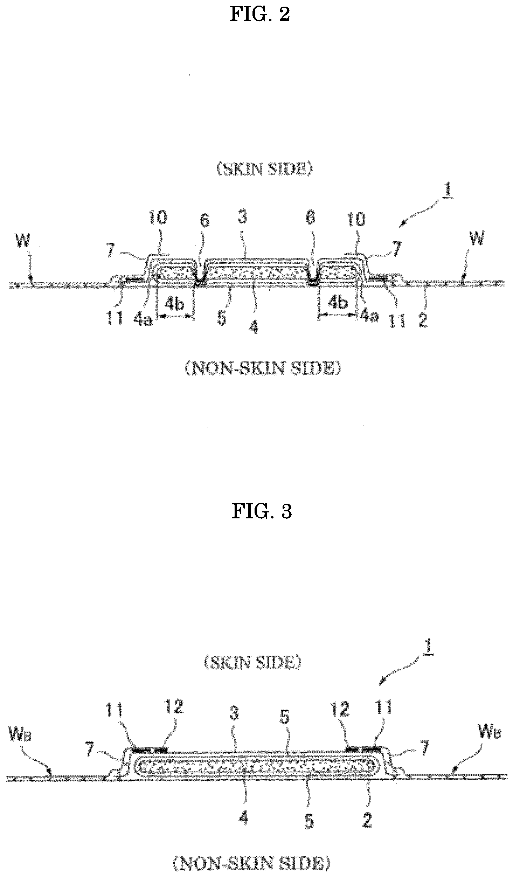

In the shown example, as shown in the cross-sectional views of FIGS. 2 and 3, the width dimension of the top-surface sheet 3 is slightly longer than the width dimension of the absorber 4, and only covers the absorber 4. At the outer side thereto, a side sheet 7 different from the top-surface sheet 3 is disposed. Specifically, a side sheet 7 formed of a nonwoven fabric material subjected to appropriate water repellent treatment or hydrophilic treatment for the purpose of preventing menstrual blood, vaginal discharge, and the like, from penetrating, or improving the touch feeling of the skin, is disposed.

As such side sheet 7, one formed by an appropriate processing method using a natural fiber, a synthetic fiber or a regenerated fiber as a raw material can be used. However, in order to eliminate a stiff feeling and to prevent a moist feeling, a soft nonwoven fabric having aeration properties with the basis weight reduced is preferably used. In particular, a nonwoven fabric having softness that is pleasant to the touch even when it is brought into contact with the skin surface is preferable. Specifically, a nonwoven fabric produced by a spunbond method by setting the basis weight at 13 to 23 g/m² is desirably used, and in order to securely prevent permeation of the body fluid, a water-repellent nonwoven fabric coated with a water repellent agent, such as a silicon-based, paraffin-based, alkylchromic chloride-based water repellent agent, or the like, is suitably used.

As shown by mark "x" in FIGS. 2 and 3, the side sheets 7 are bonded with an adhesive such as hot melt over a range from a predetermined inner position to an outer edge of the back surface sheet 2 at an outer side portion from an intermediate portion in the width direction, and flap portions in which the absorber 4 is not interposed are formed on both side portions of the absorber 4 by the laminated sheet portion of the side sheet 7 and the back surface sheet 2. In this flap portion, a pair of left and right wing flaps W, W are formed on both side portions of a portion substantially corresponding to a body fluid discharge portion H of a wearer, and back flaps W_{B}, W_{B} are formed on both back sides of the back side (buttock side) . The longitudinal section including the wing-shaped flap W corresponds to the body fluid discharge portion H of a wearer. An adhesive layer is provided on each of the wing-shaped flaps W and W and the back flaps W_{B} and W_{B} on the undergarment-contact surface side (back-surface sheet 2 side), and when the wing-shaped flaps W and W are placed to the undergarment, the wing-shaped flaps W and W are folded to the opposite side at the position of the fold line RL of the base end portion, and are wrapped around and fastened to the crotch portion of the undergarment, and the back flap WB is fastened to the inner surface of the undergarment.

On the other hand, an elastically stretchable member is not disposed in the inner side portion of the side sheet 7, so that the contraction force of the elastically stretchable member does not act on the side sheet 7 at all. Accordingly, in the natural state of the sanitary napkin 1 which is taken out from the individual packaging and is not subjected to an external force, as shown in FIGS. 2 and 3, the center portion in the width direction of the side sheet 7 is laminated in a state in which it is in contact with the upper surface of the top-surface sheet 3. When the elastically stretchable member is not provided in the side sheet 7, when the sanitary napkin 1 is worn, a wearer does not feel uncomfortable feeling because the contraction force of the elastically stretchable member does not act on the skin surface. On the other hand, since the elastically stretchable member is not provided, there is a concern that lateral leakage occurs because the side sheet does not stand up to the skin side due to contraction force of the elastically stretchable member. However, this sanitary napkin 1 prevents body fluid from leaking from the side portion by providing the following configuration.

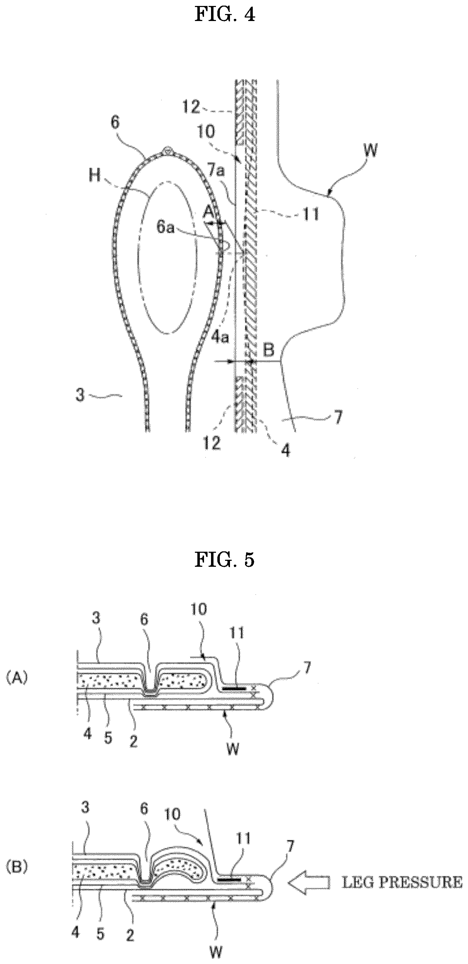

As shown in FIG. 4, this sanitary napkin 1 includes a non-adhesive portion 10, in which a center portion in the width direction of the side sheets 7 is not bonded to the absorber 4 side, in a longitudinal section including a region corresponding to a body fluid discharge portion H of a wearer; adhesive portion 11 which allows the side sheet 7 to be bonded to the outside in the width direction of the non-adhesive portion 10 to the absorber 4 side; and adhesive portions 12, 12 in which the side sheets 7 are bonded to the absorber 4 side at both ends sides in the longitudinal direction of the non-adhesive portion 10. The adhesive portion 11 formed in the outer side in the width direction of the non-adhesive portion 10 joins the side sheet 7 to the top-surface sheet 3 disposed at the lower side thereof over the entire length in the longitudinal direction of the sanitary napkin 1. Furthermore, the adhesive portion 12 formed at both ends in the longitudinal direction of the non-adhesive portion 10 joins the side sheet 7 to the top-surface sheet 3 disposed at the lower side thereof in a part from the front and back ends of the non-adhesive portion 10 to the front end or the back end of the sanitary napkin 1, respectively. In other words, the adhesive portion 11 is intermittent joining portion extending along the longitudinal direction of sanitary napkin 1, which are intermittent in the non-adhesive portion 10. In a region in which the adhesive portion 11 and the adhesive portion 12 are adjacent to each other, a boundary between these adhesive portions 11 and 12 may not be clear, and the outer side in the width direction of the non-adhesive portion 10 can be defined as the adhesive portion 11 and the inner side thereof can be defined as the adhesive portion 12 by using an extension line extending to the outer side edge in the width direction of the non-adhesive portion 10 in the longitudinal direction as a boundary.

An edge 7a at the center side in the width direction of the side sheet 7 is located to the center side in the width direction of the absorber 4. In detail, as shown in FIG. 4, when a longitudinal section including a region corresponding to a body fluid discharge portion H of a wearer includes a constricted portion 13 obtained by constricting both side edges of the absorber 4 inwardly in the width direction, the edge 7a is located to the center side in the width direction from the narrowest portion 4a of the constricted portion 13 of the absorber 4. In other words, the side edge of the absorber 4 is covered in the entire length with the center portion in the width direction of the side sheet 7.

Furthermore, in the adhesive portion 11 adjacent to the outer side in the width direction of the non-adhesive portion 10, the edge on the center side in the width direction is provided in a position overlapped to or in the vicinity at least a part of the side edge of the absorber 4. In detail, as shown in FIG. 4, the edge of the center side in the width direction of the adhesive portion 11 is provided in a position overlapped to or in the vicinity of the narrowest portion 4a of the constricted portion 13 of the absorber 4 in the longitudinal section including a region corresponding to a body fluid discharge portion H of a wearer.

With the above-mentioned configuration, in the sanitary napkin 1, as shown in FIG. 5 (A), the wing-shaped flaps W are folded such that side edge of the crotch of the undergarment is wrapped together and fixed. Then, as shown in FIG. 5(B), in a state in which when the sanitary napkin 1 is placed at crotch portion, and a leg pressure is applied by the inner portion at the leg base of a wearer from the outer side toward the inner side in the width direction, both sides of the absorbent body 4 swell toward the skin side. As the absorber is raised toward the skin side according to the swelling, the non-adhesive portion 10 of the side sheet 7 is pushed up toward the skin side, whereby the non-adhesive portion 10 of the side sheet 7 stands up toward the skin side starting from a boundary portion with the adhesive portion 11 adjacent to the outside in the width direction. Thus, the body fluid flowing to the side is blocked, and leakage from the side part can be prevented.

The center portion in the width direction of the side sheet 7 may be folded in the width direction to form a multiple sheet, but it is preferable to form a single sheet that is not folded in the width direction so that the edge of the side sheet 7 standing on the skin side is softly brought into contact with the skin surface to achieve a good wearing feeling.

In order to reliably allow the non-adhesive portion 10 of the side sheet 7 to stand up toward the skin side, it is preferable to form a constricted portion 13 obtained by inwardly constricting both side edges of the absorber 4 to the width direction in a longitudinal section including a region corresponding to a body fluid discharge portion H of a wearer. When the constricted portion 13 is provided, when the sanitary napkin 1 is worn, leg pressure is intensively applied to the narrowest part 4a of the constricted portion 13 to easily act, and both sides of the absorber 4 reliably swell toward the skin side around the narrowest part 4a of the constricted portion 13, and the non-adhesive portion 10 of the side sheet 7 reliably stands up toward the skin side.

Furthermore, when the constricted portion 13 is provided, in the outer side in the width direction of the non-adhesive portion 10, since the constricted portion 13 is located so as to extend obliquely outward on the lower layer side of the adhesive portion 11, the side sheet 7 is warped along the obliquely extending constricted portion 13, whereby the non-adhesive portion 10 on the center side in the width direction easily stands up on the skin side. In order to reliably exhibit such a warping function of the side sheet 7, the plan shape of the constricted portion 13 is preferably an arc shape.

In order to reliably allow the non-adhesive portion 10 of the side sheet 7 to stand up toward the skin side, it is preferably to provide a maximum bulging portion 6a of the compressed groove 6 and the narrowest width portion 4a of the absorber 4 in a position that substantially coincide with the longitudinal direction of the sanitary napkin 1 in the longitudinal section including a region corresponding to a body fluid discharge portion H of a wearer. Thus, the leg pressure during wearing acts intensively on the absorber part between the narrowest part 4a of the absorber 4 and the maximum bulging portion 6a of the compressed groove 6, and the absorber in this part is easily deformed, and the side sheet 7 easily stands up toward the skin side. A separation distance in the longitudinal direction of the napkin between the narrowest part 4a of the absorber 4 and the maximum bulging portion 6a of the compressed groove 6 is 10 mm or less, preferably 5 mm or less.

As shown in FIG. 4, the separation distance A in the napkin width direction between the narrowest part 4a of the absorber 4 and the maximum bulging portion 6a of the compressed groove 6 is preferably 10 to 15 mm so that the push-up effect of the side sheets 7 accompanying the swelling of the absorbent body 4 in this part can be reliably exhibited by the leg pressure during wearing.

Furthermore, as shown in FIG. 2, in the longitudinal section including a region corresponding to a body fluid discharge portion H of a wearer, the rigidity of the absorber 4 in the outer side portion 4b that is outer from the compressed grove 6 in the width direction may be set lower than the rigidity of the absorber 4 between the left and right compressed grooves 6, 6. When the rigidity of the outer side portion 4b in the width direction is made to be relatively lower, the outer side portion 4b in the width direction is easily deformed when external force is applied, and the effect of pushing up the side sheet 7 to the skin side is increased. In order to reduce the rigidity of the outer side portion 4b in the width direction, means for reducing the amount of a cotton pulp and/or a water absorbing polymer may be used.

The non-adhesive portion 10 is formed to have a length of the napkin in the longitudinal direction, which is a length including at least a region corresponding to a body fluid discharge portion H of a wearer. Specifically, as shown in FIG. 1, it is preferable that the length of the non-adhesive portion 10 is substantially equal to or slightly longer than the length of the wing-shaped flap W formed on both side portions in the longitudinal direction of the napkin. Furthermore, it is preferable that the length of the non-adhesive portion 10 is substantially equal to or slightly longer than the length in the longitudinal direction of the napkin of a portion bulging to the outer side in the width direction at both sides of the region corresponding to a body fluid discharge portion H in the compressed groove 6. In addition, it is preferable that the length of the non-adhesive portion 10 is substantially equal to or slightly longer than the length of the constricted portion 13 of the absorber 4 in the longitudinal direction. Specifically, the length of the non-adhesive portion 10 is preferably longer than the length of the wing-shaped flap W, a bulging portion in the width direction of the compressed groove 6 or the length of the constricted portion 13 by a range of 30 mm or less and preferably 20 mm or less. When the non-adhesive portion 10 has a length longer than this, the non-adhesive portion 10 does not easily stand up toward the skin side.

Furthermore, as shown in FIG. 4, the width B of the non-adhesive portion 10 is preferably not more than equal to the separation distance A in the napkin width direction between the narrowest part 4a of the absorber 4 and the maximum bulging portion 6a of the compressed groove 6 (B≤A), and specifically 3 to 15 mm, and preferably 5 to 10 mm.

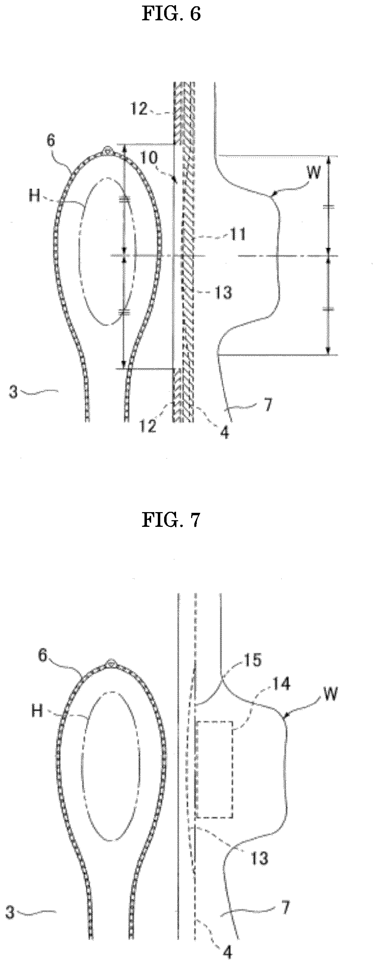

Next, the position relation between the non-adhesive portion 10 and the wing-shaped flap W is described. As shown in FIG. 6, it is preferable that the center portion in the longitudinal direction of the wing-shaped flap W and the center portion in longitudinal direction of the non-adhesive portion 10 substantially coincide with the longitudinal direction of the sanitary napkin 1. The length of the wing-shaped flap W in the longitudinal direction is the length of the napkin in the longitudinal direction at the front and back positions where the front and back outlines of the base end portion of the wing-shaped flap W start to protrude outward in the width direction. Preferably, the center line of the napkin in the width direction passing through the center portion of the wing flap W in the longitudinal direction and the center line of the napkin in the width direction passing through the center portion of the non-adhesive portion 10 in the longitudinal direction coincide with each other or are spaced apart by a range of 10 mm or less in the longitudinal direction of the napkin. Thus, when the wing-shaped flap is folded so as to wrap the side edge of the crotch portion of an undergarment and fixed to the outer surface of the undergarment, the side sheet 7 is pulled outward in the width direction, and the non-adhesive portion 10 on the center side in the width direction easily stands up toward the skin side. In order to ensure that the inner portion of the side sheet 7 is pulled outward by folding of the wing-shaped flap W, the length of the non-adhesive portion 10 in the longitudinal direction of the napkin is preferably substantially equal to or slightly longer than the length of the wing-shaped flap W in the longitudinal direction of the napkin.

A wing displacement prevention adhesive layer 14 disposed on an undergarment-contact surface of the wing flap W may be disposed substantially in the center portion of the wing flap W similar to usual sanitary napkins, but, as shown in FIG. 7, is preferably disposed along a virtual line 15 linking the side edges of the absorbent body 4 in the front and back of the region corresponding to a body fluid discharge portion H of a wearer in the longitudinal direction. In other words, as shown in the example in the drawing, in the absorber 4 having constricted portions 13 formed on both sides of a region corresponding to a body fluid discharge portion H of a wearer, the virtual line 15 including straight lines linking the side edges of the absorber 4 in the front and back of the constricted portion 13 in the longitudinal direction is drawn, and the wing displacement prevention adhesive layer 14 is disposed so that the virtual line 15 and the side edges at the inner side of the wing displacement prevention adhesive layer 14 in the width direction substantially coincide with each other. Thus, when the wing-shaped flap W is folded back so as to wrap the side edge of the crotch portion of an undergarment and fixed to the outer surface of the undergarment, the vicinity of the side edge of the absorbent body 4 is firmly fixed to the undergarment by the wing displacement prevention adhesive layer 14, the side sheet 7 can be prevented from being deflected, and the non-adhesive portion 10 can easily stand up toward the skin side. Note here that in the example shown in the drawing, the displacement prevention adhesive layer 14 is provided only at the base end portion of the wing-shaped flap W, but the displacement prevention adhesive layer 14 may be disposed in a size extending to the center portion of the wing-shaped flap W or to the outside thereof, or an adhesive layer different from the displacement prevention adhesive layer 14 may be disposed separately in the width direction.

### Reference Sings List

1... Sanitary napkin, 2... Back-surface sheet, 3... Top-surface sheet, 4... Absorber, 5... Encapsulating sheet, 6... Compressed groove, 7... Side sheet, 10... Non-adhesive portion, 11, 12... Adhesive portion, 13... Constricted portion, 14... Wing displacement prevention adhesive layer, 15... Virtual line

## Claims

1. An absorbent article comprising an absorber, and side sheets disposed on both side portions in a longitudinal direction on a skin-contact surface side over an entire length in the longitudinal direction, the side sheets not including an elastically stretchable member,
comprising:
a non-adhesive portion, in which a center portion in the width direction of each of the side sheets is not bonded to the absorber side, in a longitudinal section including a region corresponding to a body fluid discharge portion of a wearer, and
an adhesive portion in which the side sheets are bonded to the absorber side, respectively, at an outer side in the width direction and at both end sides in the longitudinal direction of the non-adhesive portion,
wherein an edge of the side sheets in the center side in the width direction is located to the center side in the width direction from a side edge of the absorber, and
the adhesive portion adjacent to the outer side in the width direction of the non-adhesive portion has an edge in the center side in the width direction provided in a position overlapped to or in the vicinity of the side edge of the absorber.

2. The absorbent article according to claim 1, wherein a constricted portion obtained by constricting both side edges of the absorber inward in the width direction is formed in the longitudinal section including a region corresponding to a body fluid discharge portion of a wearer.

3. The absorbent article according to claim 1 or 2, wherein compressed grooves dented toward a non-skin side are formed on a skin-contact surface of a main body part in which the absorber is interposed, and wherein the compressed grooves are respectively formed on both side portions of a longitudinal section including a region corresponding to a body fluid discharge portion of a wearer respectively along the longitudinal direction and in a planar shape bulging to an outer side in the width direction.

4. The absorbent article according to claim 3, wherein, in the longitudinal section including a region corresponding to a body fluid discharge portion of a wearer, a maximum bulging portion of each of the compressed grooves and a narrowest portion of the absorber are provided in a position substantially coinciding with a longitudinal direction of the absorbent article.

5. The absorbent article according to claim 3 or 4, wherein rigidity of the absorber at the outer side in the width direction from the compressed groove is set to be lower than rigidity of the absorber between left and right compressed grooves, in the longitudinal section including a region corresponding to a body fluid discharge portion of a wearer.

6. The absorbent article according to any one of claims 1 to 5, wherein a pair of wing-shaped flaps protruding to the side portions to be fixed to an undergarment are formed on both side portions in a region corresponding to a body fluid discharge portion of a wearer, a center portion in the longitudinal direction of the wing-shaped flap and a center portion in the longitudinal direction of the non-adhesive portion are provided in the position substantially coinciding with the longitudinal direction of the absorbent article.

7. The absorbent article according to any one of claims 1 to 6, wherein a pair of wing-shaped flaps protruding to the side portions to be fixed to an undergarment are formed on both side portions in a region corresponding to a body fluid discharge portion of a wearer, and a wing displacement prevention adhesive layer is provided on an undergarment-contact surface of the wing-shaped flaps, and
the wing displacement prevention adhesive layer is disposed along a virtual line linking the side edges of the absorber in a front part and a back part of a region corresponding to a body fluid discharge portion of a wearer in the longitudinal direction.
